# EUROPEAN PATENT SPECIFICATION

(11) **EP 2 575 474 B1**
(45) Date of publication and mention of the grant of the patent: **26.04.2017**
(21) Application number: 11787012.1
(22) Date of filing: 18.05.2011
(51) Int. Cl.: A61K 31/59, A61K 45/06, A61K 31/592, A61K 31/593, A61P 35/00, A61P 17/00, A61P 39/06

(54) **METHODS FOR INCREASING THE PRODUCTION OR ACTIVITY OF CATALASE**
VERFAHREN ZUR ERHÖHUNG DER PRODUKTION ODER AKTIVITÄT EINER KATALASE
PROCÉDÉS PERMETTANT D'AUGMENTER LA PRODUCTION OU L'ACTIVITÉ DE LA CATALASE

(30) Priority: 26.05.2010 US 396372 P
(43) Date of publication of application: 10.04.2013
(60) Divisional application: 14166378.1
(73) Proprietor: Nestec S.A., 1800 Vevey (CH); The Ohio State University, Columbus, OH 43201 (US)
(72) Inventor: MIDDLETON, Rondo, Paul, Creve Coeur MO 63141 (US); INPANBUTR, Nongnuch, Columbus OH 43320 (US)
(74) Representative: Rupp, Christian
(86) International application number: PCT/US2011/000891
(87) International publication number: WO 2011/149513

(56) References cited:
- US-A1- 2005 202 494
- US-A1- 2009 110 674
- MH Javanbakht ET AL: "The Effects of Vitamins E and D Supplementation on Erythrocyte Superoxide Dismutase and Catalase in Atopic Dermatitis", Iranian J Publ Health, 2 March 2010 (2010-03-02), pages 57-63, XP055080561, Retrieved from the Internet: URL:http://www.ncbi.nlm.nih.gov/pmc/articl es/PMC3468963/pdf/ijph-39-057.pdf [retrieved on 2013-09-23]
- MCCARTY ET AL: "A two-phase strategy for treatment of oxidant-dependent cancers", MEDICAL HYPOTHESES, EDEN PRESS, PENRITH, US, vol. 69, no. 3, 13 July 2007 (2007-07-13), pages 489-496, XP022140472, ISSN: 0306-9877
- JAVANBAKHT ET AL.: 'The Effects of Vitamins E and D Supplementation on Erythrocyte Superoxide Dismutase and Catalase in Atopic Dermatitis.' IRANIAN JOUMAL OF PUBLIC HEALTH, [Online] vol. 39, no. 1, 02 March 2010, pages 57 - 63 Retrieved from the Internet: <URL:http://www.sid.ir/enNEWSSID/J_pdf/8632 0100109.pdf> [retrieved on 2011-07-27]
- BROOM ET AL.: 'Effects of zinc oxide and Enterococcus faecium SF68 dietary supplementation on the performance, intestinal microbiota and immune status of weaned piglets.' RESEARCH IN VETERINARY SCIENCE, [Online] vol. 80, no. 1, February 2006, pages 45 - 54, XP005127148 Retrieved from the Internet: <URL:http://www.sciencedirect.com/science/a rticle/pii/S0034528805000755> [retrieved on 2011-07-27]

## Description

### BACKGROUND OF THE INVENTION

### Field of the Invention

The invention relates generally to measures for increasing the production of catalase and particularly to vitamin D for use in increasing the production or of catalase in animals.

The scope of the present invention is defined by the appended claims.

### Description of Related Art

Catalase is a common enzyme found in nearly all living organisms exposed to oxygen. Catalase catalyzes the decomposition of hydrogen peroxide to water and oxygen. Catalase is essentially universal in animals and plants and is found in many fungi and some anaerobic microorganisms.

In animals and many other organisms, oxidative stress is caused by an imbalance between the production of reactive oxygen and a biological system's ability to readily detoxify the reactive intermediates or easily repair the resulting damage. Cellular oxidative injury has been implicated in aging and a wide array of clinical disorders including ischemia-reperfusion injury; neurodegenerative diseases; diabetes; inflammatory diseases such as atherosclerosis, arthritis, and hepatitis; drug-induced toxicity; and cancer.

Reactive oxygen species (ROS), including the superoxide radical anion and hydrogen peroxide, are produced endogenously in response to cytokines, growth factors; G-protein coupled receptors, and physical or environmental stress. ROS function as signaling molecules to mediate various biological responses. However, an excess ROS contributes to various pathophysiologies as described. To minimize the damaging effects of excess ROS, aerobic organisms have evolved different lines of antioxidant defense. One line of defense is enzymatic, comprising catalases, peroxides, superoxide dismutase's, and glutathione-S-transferase, among others. For example, superoxide dismutase's (SOD), catalases and peroxides protect cells by directly scavenging superoxide radicals and hydrogen peroxide, converting them to less reactive species. SODs catalyze the dismutation of superoxide anion (O₂)⁻ to hydrogen peroxide (H₂O₂), and catalases and peroxides reduce H₂O₂ to water.

Thus, catalases are ubiquitous and important enzymatic components in the ROS detoxification system present in aerobic organisms. In mammals, catalase deficiencies have been associated with decreased ability to detoxify ROS in various cells and tissues, leading to or associated with a variety of detrimental conditions, including premature aging (and aging generally; see, *e.g*., Harman D., 2009, Biogerontology 10: 773-781), diabetes, cardiovascular disease, kidney disease, tumor formation, infertility and graying of hair. In connection with the latter, it has recently been determined that accumulated hydrogen peroxide and a near absence of catalase in the aging hair shaft are associated with loss of hair pigment (Wood JM et al., 2009, Faseb J. 23: 2065-2075). According to recent scientific studies, low levels of catalase may play a role in the graying process of human hair. Hydrogen peroxide is naturally produced by the body and catalase breaks it down. If there is a dip in catalase levels, hydrogen peroxide cannot be broken down. This causes the hydrogen peroxide to bleach the hair from the inside out. Scientists believe this finding may someday be incorporated into anti-graying treatments for aging hair.

The issue of lowered catalase activity in cancers has been addressed by McCarty MF et al. (2007, Med. Hypotheses 69: 489-496). The authors suggest a two-phase strategy for the treatment of oxidant-dependent cancers based on the expectation that calcitriol when used as an adjuvant will potentiate a concurrent chemotherapy, particularly high-dose ascorbate therapy. The toxicity of ascorbate has been traced to the hydrogen peroxide generated by the spontaneous reduction of molecular oxygen. However, since oxygen is required for the production of hydrogen peroxide, hypoxic tumor regions will be relatively resistant to the cytotoxicity of ascorbate. Thus, concurrent measures which boost tumor oxygenation and thus may amplify the responsiveness of many tumors to high-dose ascorbate are considered. One such measure may be calcitriol as it has been demonstrated that pretreatment with activated vitamin D (calcitriol) enhances the cytotoxic impact of hydrogen peroxide on MCF-7 breast cells and that calcitriol has a pro-oxidant effect in this cell line.

In view of the foregoing, it is clear that the development of methods and compositions to increase catalase production or activity in animals and other organisms would represent an advance in the art.

### SUMMARY OF THE INVENTION

It is, therefore, an object of the present invention to provide vitamin D for use in preventing or treating a disease or condition caused by catalase deficiency in an aging companion animal that can benefit from an increase in the production of catalase, wherein the disease or condition is acatalasemia.

It is another object of the invention to provide vitamin D as the sole active ingredient for use in preventing or treating a disease or condition caused by catalase deficiency in an aging companion animal that can benefit from an increase in the production of catalase, wherein the disease or condition is hepatoma.

It is a further object of the invention to provide a composition for use in preventing or treating a disease or condition caused by catalase deficiency in an aging companion animal that can benefit from an increase in the production of catalase, the composition comprising vitamin D and at least one additional agent increasing catalase production or activity, or reducing or eliminating reactive oxygen species, wherein the disease or condition is acatalasemia.

These objects are achieved through novel uses of vitamin D and compositions comprising vitamin D for increasing the production of catalase in an aging companion animal. The uses relate to and the compositions comprise vitamin D in an amount effective to increase the production or activity of catalase in an aging companion animal. Uses of vitamin D and compositions comprising vitamin D are also provided to treat catalase deficiency in an aging companion animal.

Other and further objects, features, and advantages of the present invention will be readily apparent to those skilled in the art.

### DETAILED DESCRIPTION OF THE INVENTION

### Definitions

The term "animal" means any animal or other organism (*e.g*., fungi) that can benefit from an increase in production or activity of catalase, including human, avian, bovine, canine, equine, feline, hicrine, lupine, murine, ovine, or porcine animals.

The term "catalase" means inclusively to enzymes of IUBMB enzyme nomenclature EC 1.11.1.6, catalyzing the reaction 2 H₂O₂ = O₂ + 2 H₂O. Other name(s) for catalase include: equilase, caperase, optidase, catalase-peroxidase and CAT. Catalase can also act as a peroxidase (EC 1.11.1.7 peroxidase) for which several organic substances, especially ethanol, can act as a hydrogen donor. A typical catalase is a tetramer of four polypeptide chains, containing four porphyrin heme (iron) groups that allow the enzyme to react with the hydrogen peroxide.

The term "companion animals" means domesticated animals such as dogs, cats, birds, rabbits, guinea pigs, ferrets, hamsters, mice, gerbils, pleasure horses, cows, goats, sheep, donkeys, pigs, and more exotic species kept by humans for company, amusement, psychological support, extrovert display, and all of the other functions that humans need to share with animals of other species.

The term "dietary supplement" means a product that is intended to be ingested in addition to the normal diet of an animal. Dietary supplements may be in any form, e.g. solid, liquid, gel, tablets, capsules, powder. Preferably they are provided in convenient dosage forms. In some embodiments they are provided in bulk consumer packages such as bulk powders or liquids. In other embodiments, supplements are provided in bulk quantities to be included in other food items such as snacks, treats, supplement bars, beverages.

The term "extending the prime" means extending the number of years an animal lives a healthy life and not just extending the number of years an animal lives, *e.g*., an animal would be healthy in the prime of its life for a relatively longer time. Thus, the "prime years" of an animal's life can extend from young adulthood ("young," as described below) into the older or "aged" population. Indeed, the prime years of an animal's life can extend essentially until the animal's death, assuming the animal is healthy and active through its older years.

The term "food" or "food composition" means a composition that is intended for ingestion by an animal, including a human. A "complete and nutritionally balanced pet food" contains all known required nutrients for the intended recipient or consumer of the food, in appropriate amounts and proportions, based for example on recommendations of recognized authorities in the field of companion animal nutrition. Such foods are therefore capable of serving as a sole source of dietary intake to maintain life or promote production, without the addition of supplemental nutritional sources. Nutritionally balanced pet food compositions are widely known and widely used in the art.

The term "gene" or "genes" means a complete or partial segment of DNA involved in producing a polypeptide, including regions preceding and following the coding region (leader and trailer) and intervening sequences (introns) between individual coding segments (exons). The term encompasses any DNA sequence that hybridizes to the complement of gene coding sequences.

The term "health and/or wellness of an animal" means the complete physical, mental, and social well being of the animal, not merely the absence of disease or infirmity.

The term "in conjunction" means that a drug, food, or other substance is administered to an animal (1) together in a composition, particularly food composition, or (2) separately at the same or different frequency using the same or different administration routes at about the same time or periodically. "Periodically" means that the substance is administered on a dosage schedule acceptable for a specific substance. "About the same time" generally means that the substance (food or drug) is administered at the same time or within about 72 hours of each other. "In conjunction" specifically includes administration schemes wherein substances such as drugs are administered for a prescribed period and compositions of the present invention are administered indefinitely.

The term "individual" when referring to an animal means an individual animal of any species or kind.

The term "long-term" administration means periods in excess of one month. Periods of longer than two, three, or four months are included. Also included are more extended periods that include longer than 5, 6, 7, 8, 9, or 10 months. Periods in excess of 11 months or 1 year are also included. Longer term use extending over 1, 2, and 3 years or more are also contemplated herein. In the case of certain animals, it is envisioned that the animal would be administered substances identified by the present methods on a regular basis. "Regular basis" as used herein refers to at least weekly administration. More frequent administration, such as twice or thrice weekly is contemplated. Also included are regimens that comprise at least once, twice, three times or more daily administration. Any dosing frequency, regardless of whether expressly exemplified herein, is considered useful. The skilled artisan will appreciate that dosing frequency will be a function of the substance that is being administered, and some compositions may require more or less frequent administration to maintain a desired biochemical, physiological or gene expression effects, namely effects including one or more of food intake, satiety, lipid metabolism, and fat utilization, and the gene expression profile associated therewith. The term "extended regular basis" as used herein refers to long-term administration of a substance on a regular basis.

The term "oral administration" means that the animal ingests, or a human is directed to feed, or does feed, the animal one or more of the substances described herein. The term "ingestion" is used herein interchangeably with the term "oral administration." Wherein a human is directed to orally administer or feed the substance, such direction may be that which instructs and/or informs the human that use of the substance may and/or will provide the referenced benefit. Such direction may be oral direction (*e.g*., through oral instruction from, for example, a physician, veterinarian, or other health professional, or radio or television media (*i.e.,* advertisement), or written direction (*e.g.,* through written direction from, for example, a physician, veterinarian, or other health professional (e*.g.*, prescriptions), sales professional or organization (*e.g*., through, for example, marketing brochures, pamphlets, or other instructive paraphernalia), written media (*e.g*., internet, electronic mail, or other computer-related media), and/or packaging associated with the substance.

The term "sample" means any animal tissue or fluid containing, *e.g.*, polynucleotides, polypeptides, antibodies, metabolites, including cells and other tissue containing DNA and RNA. Examples include adipose, blood, cartilage, connective, epithelial, lymphoid, muscle, nervous, sputum. A sample may be solid or liquid and may be DNA, RNA, cDNA, bodily fluids such as blood or urine, cells, cell preparations or soluble fractions or media aliquots thereof, chromosomes, organelles.

The term "single package" means that the components of a kit are physically associated in or with one or more containers and considered a unit for manufacture, distribution, sale, or use. Containers include, bags, boxes, bottles, shrink wrap packages, stapled or otherwise affixed components, or combinations thereof. A single package may be containers of individual food compositions physically associated such that they are considered a unit for manufacture, distribution, sale, or use.

The term "topical administration" as used herein, means the administration or application of a composition to the hair, fur, nails, claws, skin, mucosa, eye, airway, lung, or any other epithelial surface.

The term "vitamin D" means vitamin D and any of its natural or synthetic forms, derivatives, or analogs that function like vitamin D to affect the production or activity of catalase in animals. By way of background, vitamin D can be obtained from dietary sources or can be synthesized in the body. Dietary vitamin D is available in two forms: vitamin D₂ (ergocalciferol) from plant sources and vitamin D₃ (cholecalciferol) from animal sources, both of which are collectively termed vitamin D. Vitamin D in the form of vitamin D₃ can be made from 7-dehydrocholesterol (7-DHC) in the skin by exposure to ultraviolet light, mainly from light in the UVB spectrum. Vitamin D is a prohormone, converted ultimately to the active form 1α,25-dihydroxyvitamin D₃ (1,25(OH)₂D₃), also known as calcitriol. Thus, as used herein, the term "vitamin D" includes forms of vitamin D (*e.g*., vitamin D₂, vitamin D₃) that are produced by organisms and may be converted via natural or synthetic means into the biologically active form, calcitriol. The term also includes natural or synthetic analogs that mimic at least one biological activity of calcitriol. In addition to stimulating production or activity of catalase, biological activities of calcitriol and/or its precursors include, binding to the vitamin D binding protein (precursors), binding to vitamin D receptors (calcitriol) and subsequent signal transduction, and stimulating calcium absorption.

The term "virtual package" means that the components of a kit are associated by directions on one or more physical or virtual kit components instructing the user how to obtain the other components, *e.g.,* in a bag containing one component and directions instructing the user to go to a website, contact a recorded message, view a visual message, or contact a caregiver or instructor to obtain instructions on how to use the kit.

"Young" refers generally to an individual in young adulthood, *i.e.*, matured past puberty or adolescence, as would be defined by species, or by strain, breed or ethnic group within a species, in accordance with known parameters. "Aged" or "old," as used herein, refers to an individual who is physically or chronologically within the last 30% of its average life expectancy, as determined by species, or by strain, breed or ethnic group within a species, in accordance with known parameters.

As used herein, ranges are used herein in shorthand, so as to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "a compound" or "a method" includes a plurality of such "compounds" or "methods." Similarly, the words "comprise", "comprises", and "comprising" are to be interpreted inclusively rather than exclusively. Likewise the terms "include", "including" and "or" should all be construed to be inclusive, unless such a construction is clearly prohibited from the context.

The terms "comprising" or "including" are intended to include embodiments encompassed by the terms "consisting essentially of" and "consisting of". Similarly, the term "consisting essentially of" is intended to include embodiments encompassed by the term "consisting of".

All percentages expressed herein are by weight of the composition on a dry matter basis unless specifically stated otherwise. The skilled artisan will appreciate that the term "dry matter basis" means that an ingredient's concentration in a composition is measured after any free moisture in the composition is removed.

Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

### The Invention

In one aspect, the invention provides vitamin D and composition comprising vitamin D for increasing the production of catalase in animals. The invention is based in part upon the discovery that vitamin D increases the production of catalase and upregulates the expression of genes associated with catalase production in animals when administered in the proper amounts. In particular, as described in greater detail in the examples, calcitriol and certain vitamin D analogs increase catalase production, increase catalase activity, and up-regulate the expression of genes encoding catalase.

The animal is an animal that could benefit from an increase in production of catalase, *e.g.*, any aerobic animal that breathes air or who lives in an oxygen-containing environment. More particularly, the animal is a companion animal such as a canine or feline, including dogs or cats.

Such an animal is an aging animal, that is or is at risk of being a catalase deficient animal, or an animal suffering from a disease or condition related to catalase deficiency, *i.e.* Acatalasemia or hepatoma.

The vitamin D is a vitamin D that increases the production of catalase in an animal, namely calcitriol (1a,25-dihydroxyvitamin D3), 25(OH) vitamin D₃, 20-Epi-1α,25(OH)₂D₃, or 1α,24(R)(OH)₂D₃ . Combinations of two or more of the aforementioned vitamin D are also included in the invention. In certain embodiments, vitamin D forms or analogs that possess selected biological activities of calcitriol, but that are reduced or lacking in other biological activities of calcitriol, are used. For example, vitamin D forms or analogs that bind to vitamin D receptors, but that do not stimulate calcium absorption, may be suitable for use in various embodiments of the invention.

The amount of vitamin D needed to increase catalase production or activity in an animal will vary according to the form of vitamin D, the species of animal, the biological condition of the animal, the tissue(s) being treated, and other parameters well understood by a skilled artisan, *e*.*g*., a medicinal chemist or biologist. A dosage of from about 1 nanogram per kilogram (ng/kg) of body weight to about 10 milligrams per kilogram (mg/kg) of body weight is suitable for most uses, preferably from about 10 ng/kg body weight to about 5 mg/kg body weight, more preferably from about 100 ng/kg body weight to about 5 mg/kg body weight, most preferably from about 1 microgram per kilogram (µg/kg) body weight to about 1 mg/kg body weight. In various embodiments, vitamin D in administered in amounts of about 1, 10, 50, 100, 250, and 500 ng/kg body weight; about 1, 10, 50, 100, 250, and 500 µg/kg body weight; or about 1, 2, 4, 6, 8, and 10 mg/kg body weight. When useful, the vitamin D is administered in amounts greater than 10 mg/kg body weight. A conversion of these dosage ranges to International Units (IU) may be made using the formula 40 IU = 1 µg of vitamin D. Dosages may also be calculated based on the concentration of vitamin D to be delivered to the target cells or tissues. Suitable concentrations comprise at least about 10⁻¹⁵ M, or at least about 10⁻¹⁴ M, or at least about 10⁻¹³ M, or at least about 10⁻¹² M, or at least about 10⁻¹¹ M, or at least about 10⁻¹⁰ M, or at least about 10⁻⁹ M, or at least about 10⁻⁸ M, or at least about 10⁻⁷ M, or at least about 10⁻⁶ M, or at least about 10⁻⁵ M of vitamin D being made available for uptake by target cells.

In one embodiment, the vitamin D is administered orally, *e.g.,* in a composition formulated for oral administration to the animal such as a tablet, drink, medication, or food composition. Administration in accordance with the methods can be on an as-needed or as-desired basis of varying or regular frequency. A goal of regular ingestion is to provide the animal with a regular and consistent dose of the composition or the direct or indirect metabolites that result from such ingestion. Such regular and consistent dosing will tend to create constant blood levels of the components of the compositions or their direct or indirect metabolites. Thus, regular administration can be once monthly, once weekly, once daily, or more than once daily. Similarly, administration can be every other day, week, or month, every third day, week, or month, every fourth day, week, or month. Administration can be multiple times per day. When utilized as a supplement to ordinary dietetic requirements, the composition may be administered directly to the animal. The compositions can alternatively be contacted with, or admixed with, daily feed or food, including a fluid, such as drinking water, or an intravenous connection for an animal that is receiving such treatment. When utilized as a daily feed or food, administration will be well known to those of ordinary skill.

Administration can also be carried out as part of a dietary regimen for the animal. For example, a dietary regimen may comprise causing the regular ingestion by the animal of vitamin D or a vitamin D composition described herein in an amount effective to increase catalase production or activity in the animal.

Subject of the administration of vitamin D, including administration as part of a dietary regimen, is an aging animal. An animal that has reached about 35% of its projected lifespan is generally suitable. In certain embodiments administration begins, for example, on a regular or extended regular basis, when the animal has reached more than about 30%, 40%, or 50% of its projected or anticipated lifespan. In some embodiments, the animal has attained 40, 45, or 50% of its anticipated lifespan. In yet other embodiments, the animal is older having reached 60, 66, 70, 75, or 80% of its likely lifespan. A determination of lifespan may be based on actuarial tables, calculations, estimates, and may consider past, present, and future influences or factors that are known to positively or negatively affect lifespan. Consideration of species, gender, size, genetic factors, environmental factors and stressors, present and past health status, past and present nutritional status, may also influence or be taken into consideration when determining lifespan.

In various embodiments, vitamin D is administered in a food composition such as a pet food composition, or a dietary supplement. For instance, the composition can be a food composition further comprising about 15% to about 50% protein, about 5% to about 40% fat, about 5% to about 10% ash content, and having a moisture content of about 5% to about 20%. As described in greater detail below, the composition can contain additional ingredients, including vitamins, minerals, probiotics, prebiotics, or a combination thereof. The uses described herein may also employ pharmaceutical or nutraceutical compositions, formulated for administration by any selected route, as described in greater detail below.

In other embodiments, vitamin D is administered in conjunction with one or more probiotics, prebiotics, or combinations thereof to benefit the animals, *e.g.*, treat catalase deficiency in an animal.

As disclosed herein without being part of the invention, catalase production or activity is increased in target epithelial cells and tissues through the topical administration of vitamin D, preferably in a composition containing the vitamin D. Cells and tissues that can be targeted in this manner include skin, hair, fur, nails, claws, oral, anal or vaginal mucosa, gastrointestinal mucosa/epithelium, ocular surfaces, otolaryngeal and airway surfaces, including bronchial surfaces and lung alveolar surfaces. Such methods can be used to supplement a catalase deficiency in those cells and tissues, which exacerbate damage caused by an accumulation of hydrogen peroxide. For example, topical application of vitamin D to the hair and scalp could reduce or prevent graying of the hair resulting from accumulated hydrogen peroxide and catalase deficiency in tissue associated with growing hair.

The vitamin D can be administered in conjunction with one or more additional agents useful for increasing catalase production or activity, or for reducing or eliminating reactive oxygen species in the animal. For example, the additional agent(s) can be administered before, during, or after the administration of vitamin D. If the additional agents are administered together with the vitamin D, they may be administered together or separately, *e.g*., within a single composition, or in separate compositions.

The additional agents are one or more antioxidants. The antioxidants can be any antioxidant suitable for administration to an animal. Antioxidants are well known in the art, particularly the art of food technology and food formulation. Natural antioxidant compounds include vitamins (such as A, C and E, and derivative, conjugates, or analogs thereof), as well as plant extracts, including extracts from fruit, vegetables, herbs, seeds, and other types and/or parts of plants. Compounds such as α-lipoic acid, chlorophyll and derivatives thereof, glutathione, ubiquinols (*e.g.,* coenzyme Q10), carotenoids (*e.g.,* lycopene), flavonoids, phenolic acids and polyphenols, and pycnogenol are known to be excellent antioxidants. Some examples of plant sources of antioxidants include those from fruits such as berries (cherry, blackberry, strawberry, raspberry, crowberry, blueberry, bilberry/wild blueberry, black currant), pomegranate, grape, orange, plum, pineapple, kiwi fruit, and grapefruit; those from vegetables including kale, chili pepper, red cabbage, peppers, parsley, artichoke, Brussels sprouts, spinach, lemon, ginger, garlic, and red beets; those from dry fruits like apricots, prunes, and dates; from legumes including broad beans, pinto beans, and soybeans. Also nuts and seeds such as pecans, walnuts, hazelnuts, ground nut, and sunflower seeds; cereals such as barley, millet, oats, and corn. Many natural antioxidants are also available from a wide variety of spices including cloves, cinnamon, rosemary, and oregano. Less widely known sources of antioxidants include Ginkgo biloba, and tropical plants such as uyaku, and carica papaya. Antioxidant properties of various teas and green tea, as well as fermented products such as red wine, have become of great interest in recent years and such would be suitable for use herein. Selenium is an excellent oxygen scavenger and works well, especially with vitamin or related tocopherol compounds. Synthetic dietary antioxidants include butylated hydroxyanisole (BHA) and butylated hydroxytoluene (BHT) which are commonly used in food products. Any of the foregoing, alone or in combination, are suited for use herein, as are combinations of natural and synthetic antioxidants. In one embodiment, the antioxidants comprise astaxanthin alone or in combination with other antioxidants.

Further disclosed herein is a method for preventing or treating a disease or condition caused by catalase deficiency in animals. This method comprises (1) determining that an animal is or is likely to be catalase deficient and (2) administering vitamin D to the animal in an amount effective for increasing the production or activity of catalase in the animal, thereby preventing or treating the disease or condition. The condition is any condition caused by catalase deficiency, e.g., Acatalasemia or hepatoma.

In another aspect, the invention provides compositions useful for increasing the production and, optionally, activity of catalase in an animal. These compositions comprise vitamin D and at least one additional agent for increasing catalase production or activity, or for reducing or eliminating reactive oxygen species in the animal.

The vitamin D in the composition is in one embodiment D, calcitriol (1a,25-dihydroxyvitamin D₃). Other suitable forms of vitamin D are 25(OH) vitamin D₃, 20-Epi-1α,25(OH)₂D₃, and 1α,24(R)(OH)₂D₃ . Combinations of two or more of the aforementioned vitamin D are also included in the invention. In certain embodiments, vitamin D forms or analogs that possess selected biological activities of calcitriol, but that are reduced or lacking in other biological activities of calcitriol, are used. For example, vitamin D forms or analogs that bind to vitamin D receptors, but that do not stimulate calcium absorption, may be suitable for use in various embodiments of the invention.

The amount of vitamin D in the composition is any amount needed to increase catalase production in an animal. The amount will vary according to the form of vitamin D, the species of animal, the biological condition of the animal, the tissue(s) being treated, and other parameters well understood by a skilled artisan, *e.g*., medicinal chemist or biologist. A composition formulated to deliver a dosage of between about 1 µg/kg body weight and about 1,000 µg/kg body weight is suitable for most uses. More particularly a dosage of at least about 1 µg/kg body weight, or at least about 5 µg/kg body weight, or at least about 10 µg/kg body weight, or at least about 20 µg/kg body weight, or at least about 30 µg/kg body weight, or at least about 40 µg/kg body weight, or at least about 50 µg/kg body weight, or at least about 100 µg/kg body weight, or at least about 200 µg/kg body weight, or at least about 300 µg/kg body weight, or at least about 400 µg/kg body weight or at least about 500 µg/kg body weight may be utilized. A conversion of these dosage ranges to International Units (IU) may be made using the formula 40 IU = 1 µg of vitamin D. Compositions may also be formulated to deliver dosages calculated based on the concentration of vitamin D to be delivered to the target cells or tissues. Suitable concentrations comprise at least about 10⁻¹⁰ M, or at least about 10⁻⁹ M, or at least about 10⁻⁸ M, or at least about 10⁻⁷ M, or at least about 10⁻⁶ M, or at least about 10⁻⁵ M of vitamin D being made available for uptake by target cells.

The additional agents are any agents useful in increasing production or activity of catalase, or increasing the production or activity of any antioxidant system in the body or for detoxifying one or more reactive oxygen species. In one embodiment, the additional agents are one or more antioxidants. The antioxidants are any antioxidant suitable for administration to an animal. Antioxidants are well known in the art, particularly the art of food technology and food formulation. Natural antioxidant compounds include vitamins (such as A, C and E, and derivative, conjugates, or analogs thereof), as well as plant extracts, including extracts from fruit, vegetables, herbs, seeds, and other types and/or parts of plants. Compounds such as α-lipoic acid, chlorophyll and derivatives thereof, glutathione, ubiquinols (*e.g*., coenzyme Q10), carotenoids (*e.g.,* lycopene), flavonoids, phenolic acids and polyphenols, and pycnogenol are known to be excellent antioxidants. Some examples of plant sources of antioxidants are listed above. Selenium is an excellent oxygen scavenger and works well, especially with vitamin or related tocopherol compounds. Synthetic dietary antioxidants include butylated hydroxyanisole (BHA) and butylated hydroxytoluene (BHT) which are commonly used in food products. Any of the foregoing, alone or in combination, are suited for use herein, as are combinations of natural and synthetic antioxidants. In one embodiment, the antioxidants comprise astaxanthin alone or in combination with other antioxidants.

The compositions may further comprise substances such as minerals, other vitamins, salts, functional additives including, for example, palatants, colorants, emulsifiers, antimicrobial or other preservatives. Minerals that may be useful in such compositions include, for example, calcium, phosphorous, potassium, sodium, iron, chloride, boron, copper, zinc, magnesium, manganese, iodine, selenium. Examples of additional vitamins useful herein include such fat soluble vitamins as A, E, and K. Inulin, amino acids, enzymes, coenzymes, may be useful to include in various embodiments.

The compositions can be formulated as a pet food composition, or dietary supplement. Such compositions include foods intended to supply the necessary dietary requirements for an animal, animal treats (*e.g*., biscuits), or dietary supplements. The compositions may be a dry composition (*e.g*., kibble), semi-moist composition, wet composition, or any mixture thereof. In another embodiment, the composition is a dietary supplement such as a gravy, drinking water, beverage, yogurt, powder, granule, paste, suspension, chew, morsel, treat, snack, pellet, pill, capsule, tablet, or any other suitable delivery form. The dietary supplement can comprise a high concentration of the vitamin D. This permits the supplement to be administered to the animal in small amounts, or in the alternative, can be diluted before administration to an animal. The dietary supplement may require admixing, or preferably be admixed with water or other diluents prior to administration to the animal.

In one embodiment, the compositions are refrigerated or frozen compositions. In another embodiment, the vitamin D is pre-blended with the other components to provide the beneficial amounts needed. In yet other embodiments, the vitamin D is used to coat a food, snack, pet food composition, or pet treat. In one embodiment, the vitamin D is added to the composition just prior to offering it to the animal, e.g., using a sprinkled powder or a mix.

The compositions can optionally comprise one or more supplementary substances that promote or sustain general health, as would be appreciated by the skilled artisan.

In various embodiments, pet food or pet treat compositions comprise from about 15% to about 50% crude protein. The crude protein material may comprise vegetable proteins such as soybean meal, soy protein concentrate, corn gluten meal, wheat gluten, cottonseed, and peanut meal, or animal proteins such as casein, albumin, and meat protein. Examples of meat protein useful herein include pork, lamb, equine, poultry, fish, and mixtures thereof.

The compositions may further comprise from about 5% to about 40% fat. The compositions may further comprise a source of carbohydrate. The compositions may comprise from about 15% to about 60% carbohydrate. Examples of such carbohydrates include grains or cereals such as rice, corn, milo, sorghum, alfalfa, barley, soybeans, canola, oats, wheat, and mixtures thereof. The compositions may also optionally comprise other materials such as dried whey and other dairy by-products.

In some embodiments, the ash content of the composition ranges from less than 1% to about 15%, preferably from about 5% to about 10%.

The moisture content can vary depending on the nature of the composition. In a preferred embodiment, the composition is a complete and nutritionally balanced pet food. In this embodiment, the pet food may be a "wet food", "dry food", or food of intermediate moisture content. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15% or 20%, and therefore is presented, for example, as small biscuit-like kibbles. In one presently preferred embodiment, the compositions have moisture content from about 5% to about 20%. Dry food products include a variety of foods of various moisture contents, such that they are relatively shelf-stable and resistant to microbial or fungal deterioration or contamination. Also preferred are dry food compositions which are extruded food products, such as pet foods, or snack foods for either humans or companion animals.

The compositions may also comprise one or more fiber sources. The term "fiber" includes all sources of "bulk" in the food whether digestible or indigestible, soluble or insoluble, fermentable or nonfermentable. Preferred fibers are from plant sources such as marine plants but microbial sources of fiber may also be used. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, fructooligosaccharide, pectin, short chain oligofructose, mannanoligofructose, soy fiber, arabinogalactan, galactooligosaccharide, arabinoxylan, or mixtures thereof.

Alternatively, the fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may also be incorporated into the composition to aid in the enhancement of the benefit provided by the present invention to the immune system of an animal.

In various embodiments, the compositions further comprise one or more probiotics. In various embodiments, the compositions contain (1) one or more killed or inactivated probiotics, (2) components of the killed or inactivated probiotics that promote health benefits similar to or the same as the live probiotics, or (3) combinations thereof. The probiotics or their components can be integrated into the compositions (*e.g*., uniformly or non-uniformly distributed in the composition) or applied to the food compositions (*e.g*., topically applied with or without a carrier). In one embodiment, the probiotics are encapsulated in a carrier. Typical probiotics include, probiotic strains selected from *Lactobacilli, Bifidobacteria,* or *Enterococci, e*.*g*., *Lactobacillus reuteii, Lactobacillus acidophilus, Lactobacillus animalis, Lactobacillus ruminis, Lactobacillus johnsonii, Lactobacillus casei, Lactobacillus paracasei, Lactobacillus rhamnosus, Lactobacillus fermentum,* and *Bifidobacterium sp., Enterococcus faecium* and *Enterococcus sp.* In some embodiments, the probiotic strain is selected from the group consisting of *Lactobacillus reuteri* (NCC2581; CNCMI-2448), *Lactobacillus reuteri* (NCC2592; CNCM I-2450), *Lactobacillus rhamnosus* (NCC2583; CNCMI-2449), *Lactobacillus reuteri* (NCC2603; CNCM I-2451), *Lactobacillus reuteri* (NCC2613; CNCM I-2452), *Lactobacillus acidophilus* (NCC2628; CNCM I-2453), *Bifidobacterium adolescentis* (*e.g.* NCC2627), *Bifidobacterium sp.* NCC2657 or *Enterococcus faecium* SF68 (NCIMB 10415). The compositions contain probiotics in amounts sufficient to supply from about 10⁴ to about 10¹² cfu/animal/day, preferably from 10⁵ to about 10" cfu/animal/day, most preferably from 10⁷ to 10¹⁰ cfu/animal/day. When the probiotics are killed or inactivated, the amount of killed or inactivated probiotics or their components should produce a similar beneficial effect as the live microorganisms. Many such probiotics and their benefits are known to skilled artisans, *e.g*., EP1213970B1, EP1143806B1, US7189390, EP1482811B1, EP1296565B1, and US6929793.

In various embodiments, the comestible compositions further comprise one or more prebiotics, *e.g.,* fructo-oligosaccharides, gluco-oligosaccharides, galacto-oligosaccharides, isomaltooligosaccharides, xylo-oligosaccharides, soybean oligosaccharides, lactosucrose, lactulose, and isomaltulose. In certain embodiments, the prebiotic is chicory, chicory root extract, aleurone, inulin, or combinations thereof. The prebiotics are integrated into the compositions as described herein, *e.*g., uniformly or non-uniformly distributed in the composition) or applied to the food compositions (*e.g*., topically applied with or without a carrier. Generally, prebiotics are administered in amounts sufficient to positively stimulate the healthy microflora in the gut. Typical amounts are from about 1 to about 10 grams per serving or from about 5 to about 40% of the recommended daily dietary fiber for an animal.

In various embodiments, the comestible compositions further comprise a combination of one or more probiotics and one or more prebiotics.

The compositions and dietary supplements may be specially formulated for the intended recipients or consumers, i.e. for older animals. For example, a composition adapted for aging animals can be prepared. In general, specialized compositions will comprise energy and nutritional requirements appropriate for animals at different stages of development or age.

Certain aspects of the invention are preferably used in combination with a complete and balanced food. According to certain embodiments provided herein, the compositions comprising the vitamin D are preferably used with a high-quality commercial food. As used herein, "high-quality commercial food" refers to a diet manufactured to produce the digestibility of the key nutrients of 80% or more, as set forth in, for example, the recommendations of the National Research Council above for dogs, or in the guidelines set forth by the Association of American Feed Control Officials. Similar high nutrient standards would be used for other animals.

In another embodiment, the invention provides pharmaceutical compositions comprising a composition of the present invention as described above, and one or more pharmaceutically acceptable carriers, diluents, or excipients. Generally, pharmaceutical compositions are prepared by admixing a compound or composition with excipients, buffers, binders, plasticizers, colorants, diluents, compressing agents, lubricants, flavorants, moistening agents, including other ingredients known to skilled artisans to be useful for producing pharmaceuticals and formulating compositions that are suitable for administration to an animal as pharmaceuticals. The pharmaceutical composition can be formulated for any mode of administration. In one embodiment, the pharmaceutical composition is formulated for oral administration as described above. In another embodiment, the composition is formulated for topical administration. Suitable topical formulations may include solutions, emulsions, creams, ointments, gels, liposomes, biodegradable microparticles, and other such delivery vehicles as would be well understood by the pharmaceutical chemist.

Further disclosed herein are kits useful for administering a composition comprising one or more forms of vitamin D to an animal. The kits include, in separate containers in a single package or in separate containers in a virtual package, as appropriate for the kit component, (a) one or more forms of vitamin D, (b) one or more of (1) one or more other ingredients suitable for consumption by an animal; (2) one or more other agents for increasing production or activity of catalase, (3) one or more antioxidants; (4) one or more agents for reducing reactive oxygen species; (5) one or more probiotics; (6) one or more prebiotics; (7) one or more diagnostic devices suitable for determining whether an animal can benefit from compositions and methods for increasing production or activity of catalase; (8) instructions for how to combine or prepare the vitamin D, and any other ingredients provided in the kit for administration to an animal; (9) instructions for how to use the combined kit components, prepared kit components, or other kit components for the benefit of an animal; and (10) a device for administering the combined or prepared kit components to an animal.

Further disclosed herein without being part of the invention are methods for identifying an agent or regimen that increases the production or activity of catalase in cells or animals, particularly in a manner similar to that of vitamin D, *e.g.,* a vitamin D mimetic. The methods comprise (a) determining a first gene expression profile by measuring transcription or translation products of genes encoding catalase, manganese superoxide dismutase, copper-zinc superoxide dismutase, and, optionally, one or more of glutathione peroxidase, parathyroid hormone-related protein, transforming growth factor-beta 1, and vitamin D receptor, in a cell sample in the absence of the agent or regimen; (b) determining a second gene expression profile by measuring transcription or translation products of genes encoding catalase, manganese superoxide dismutase, copper-zinc superoxide dismutase, and, optionally, one or more of glutathione peroxidase, parathyroid hormone-related protein, transforming growth factor-beta 1, and vitamin D receptor, in a cell sample the presence of the agent or regimen, and (c) comparing the first gene expression profile with the second gene expression profile, wherein an increase in the catalase transcription or translation products, but not in the transcription or translation products of the other genes, in the second gene expression profile indicates that the agent or regimen is likely to increase the production or activity of catalase in a cell, in a manner similar to that of vitamin D.

Assays for measuring changes in gene expression or enzyme activity are well known to the skilled artisan. Typically, gene expression assays will measure changes in transcription or translation products of selected genes (1) by measuring transcribed RNA, *e.g*., through a hybridization or amplification assay using nucleic acid probes or primers, or (2) by measuring protein, *e.g*., through an immunological binding assay, such as a Western blot or ELISA assay, using antibody probes. Moreover, assays for measuring activity of one or more of the above-listed enzymes are also well known in the art. An assay for measuring catalase activity is described in Example 3 herein.

Methods of this type may further comprise comparing at least the second gene expression profile with a reference gene expression profile obtained by measuring transcription or translation products of one or more genes encoding the aforementioned proteins in the presence of a reference substance, such as calcitriol, which has been demonstrated to increase the production or activity of catalase, *i.e.,* by upregulating catalase gene expression.

The comparison between the test gene expression profile and the reference profile can be relatively contemporaneous, *i.e.*, cells or tissues are assayed side by side. Alternatively, however, the reference used for comparison can be based upon data previously obtained using the method.

The method may be performed on a population of cultured cells. In one embodiment, transcription or translation products of the cells are measured in the presence or absence of a test compound or a reference compound, *i.e.*, by detection with a probe or amplification primer that specifically hybridizes with mRNA encoded by the above-listed genes, or by detection with an antibody that specifically binds to a protein encoded by the genes. Alternatively, reporter genes can be utilized in a recombinant system. In either embodiment, the host cells can be mammalian cell lines, preferably originating from a human or a companion animal, such as a canine or feline. Alternatively, the methods can be performed on cells or tissues freshly isolated from a subject.

The method may be performed on animals. Typically, a test compound or test regimen (dietary, exercise) is administered to a subject and the gene expression profile in a selected tissue of the subject is analyzed to determine the effect of the test compound on transcription or translation of the aforementioned genes. Gene expression can be analyzed in situ or ex vivo to determine the effect of the test compound. In another embodiment, a test compound or regimen is administered to a subject and the activity of a protein expressed from a gene is analyzed in situ or ex vivo according to any means suitable in the art to determine the effect of the test compound on the activity of the proteins of interest. In addition, where a test compound is administered to a subject, the physiological, systemic, and physical effects of the compound, as well as potential toxicity of the compound can also be evaluated.

Further disclosed herein without being part of the invention is a substance or regimen identified by the methods for identifying an agent or regimen that increases the production or activity of catalase as likely to increase the production or activity of catalase when administered to an individual. Due to the manner in which they are identified, such substances should act as vitamin D mimetics, at least with respect to increasing catalase production in a manner similar to that of vitamin D.

Further disclosed herein without being part of the invention are kits useful for identifying an agent or regimen that increases the production or activity of catalase in cells, in a manner similar to that of vitamin D. This type of kit includes: (1) reagents suitable for measuring transcription or translation products of two or more genes encoding catalase, manganese superoxide dismutase, copper-zinc superoxide dismutase, and, optionally, one or more of glutathione peroxidase, parathyroid hormone-related protein, transforming growth factor-beta 1, and vitamin D receptor, and (2) instructions for how to use the measurements of expression of the genes to determine if an agent or regimen increases the production or activity of catalase in cells, in a manner similar to that of vitamin D. Such kits may further comprise one or more of: (1) a sample of vitamin D; and (2) information communicating the level of expression of the genes encoding catalase, manganese superoxide dismutase, copper-zinc superoxide dismutase, and, optionally, one or more of glutathione peroxidase, parathyroid hormone-related protein, transforming growth factor-beta 1, and vitamin D receptor, expected for cells exposed to vitamin D.

Further disclosed herein without being part of the invention are a means for communicating information about or instructions for one or more of (1) benefits of increasing catalase production or activity in an animal; (2) admixing one or more of: forms of vitamin D, other agents for increasing production or activity of catalase, antioxidants, agents for reducing reactive oxygen species, probiotics, prebiotics, and other ingredients suitable for consumption by an animal, to produce compositions for increasing the production or activity of catalase in an animal, (3) instructions for administering the compositions to an animal, (4) information for determining if an animal will benefit from increased catalase production or activity, and (5) information pertaining to screening forms of vitamin D for their ability to increase production or activity of catalase, the means for communicating comprising one or more of a physical or electronic document, digital storage media, optical storage media, audio presentation, audiovisual display, or visual display containing the information or instructions. These communication means can include one or more of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof. The communication means is useful for, among other things, instructing on the benefits of using the present invention and for providing contact information for a consumer or user of the invention to obtain help in using or interpreting the results from the invention.

Further disclosed herein without being part of the invention is a package comprising a composition of the present invention and a label affixed to the package containing a word or words, picture, design, acronym, slogan, phrase, or other device, or combination thereof, that indicates that the contents of the package contains a composition suitable for increasing the amount or activity of catalase in an animal. Typically, such a device comprises the words "improves antioxidant response", "alleviates oxidative stress", "helps maintain animals in their prime years", "promotes health and wellness" or an equivalent expression printed on the package. Any package or packaging material suitable for containing the composition is useful in the invention, *e.g*., a bag, box, bottle, can, pouch, manufactured from paper, plastic, foil, metal, In a preferred embodiment, the package contains a food composition adapted for a particular animal such as a human, canine or feline, as appropriate for the label, preferably a companion animal food composition.

In another aspect, the invention provides for use of vitamin D to prepare a medicament. The medicament is useful for increasing the production or activity of catalase in an animal. In various embodiments, the medicament further comprises one or more additional agents useful for increasing catalase production or activity, or for reducing or eliminating reactive oxygen species in the animal. Generally, medicaments are prepared by admixing vitamin D with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal. In various embodiments, the medicament further comprises a probiotic, prebiotic, or combination thereof.

In another aspect, the invention provides methods for manufacturing a composition comprising at least one vitamin D and one or more additional agents useful for increasing catalase production or activity, or for reducing or eliminating reactive oxygen species in the animal. The methods comprise admixing vitamin D and one or more additional agents. The agents are any compound or other substance useful for increasing catalase production or activity, or for reducing or eliminating reactive oxygen species in the animal. When the composition is a food, the composition may comprise one or more other ingredients suitable for consumption by an animal, *e.g*., protein, fat, carbohydrate, fiber, vitamins, minerals, probiotics, prebiotics, and the like. Alternatively, the methods comprise applying the vitamin D, agents, and other ingredients if desired, separately or in any combination onto a food, *e.g.,* as a coating or topping. The vitamin D can be added at any time during the manufacture and/or processing of the food. This includes, for example, admixing the vitamin D and other selected ingredients as part of the core formulation of the "body" of the food composition or applying them as a coating, *i.e*., primarily to the surface of the food after its manufacture. The compositions can be made according to any method suitable in the art.

### EXAMPLES

The invention can be further illustrated by the following examples, although it will be understood that these examples are included merely for purposes of illustration and are not intended to limit the scope of the invention unless otherwise specifically indicated.

### Example 1

Canine bladder transitional cell carcinoma (cbTCC) cells were grown in DMEM medium supplemented with 5% FBS, 5% newborn calf serum, and penicillin-streptomycin (GIBCO BRL, Grand Island, NY, USA) at 37°C in a humidified atmosphere of 5% CO2. Cells were plated at 50,000 cells/ml in 6-well culture plates (Coming Incorporated Life Sciences, Acton, MA, USA) and incubated for 24 hours before treatment. For western blot analysis, cells were treated with calcitriol (1 α,25-dihydroxyvitamin D₃) at 10⁻⁷M and 10⁻⁹M, and seocalcitol (EB1089, 1α,25-dihydroxy-22,24-diene-24,26,27-trihomovitamin D) at 10⁻⁹M for 24 hours. For enzyme activity analysis, cells were treated with calcitriol at 10⁻⁷M and 10⁻⁹M, seocalcitol at 10⁻⁹M and analog V (1α,25-dihydroxy-16-ene-23-yne-vitamin D₃) at 10⁻⁹M for 24 hours. For gene expression analysis, cells were treated with calcitriol at 10⁻⁷M and 10⁻⁹M, and seocalcitol at 10⁻⁷ M and 10⁻⁹ M for 24 hours. Treatments were done in at least triplicates. The final concentration of vehicle (ethanol) in all controls was less than 0.1% of media.

### Example 2

Harvested cells were homogenized, and analyzed for the production of manganese superoxide dismutase (MnSOD), copper/zinc superoxide dismutase (Cu/ZnSOD), catalase (CAT), and glutathione peroxidase (GPX). Cell lysates were collected after centrifugation, and total protein was quantitated. Equivalent amounts of total protein (20 or 40 µg) were resolved on precast 10% polyacylamide gels and electronically transferred to nitrocellulose membranes. Transblotted membranes were blocked overnight at 4°C with the addition of 5% skim milk in Tris-buffered saline (TBS). The membranes were cut at the molecular weight of 37 kD (green band) and 25 kD (pink band). The top part of the membranes were stained for CAT, GPX, and ß-actin, and the bottom part for MnSOD and Cu/ZnSOD. The primary antibodies for specific proteins were used at the following dilutions: Rabbit-anti-human MnSOD at 1:2000 (Upstate Biotech); Sheep-anti-human Cu/ZnSOD at 1:2000 (Calbiochem); Sheep-anti-human catalase at 1:1000 (Bidesign); and Sheep-anti-human GPX at 1:500 (Biodesign). Secondary antibodies are their respective IgG-HRP conjugates used at 1:2000. Data were reported as intensity of protein bands relative to the control (normalized to β-actin levels), considering control as 100 percent. CAT, MnSOD and Cu/ZnSOD, n=7; GPX, n=4. Prior to transforming data as percent of control, a one-tail paired t-test assuming equal variance was performed to determine significance. Results are shown in Table 1.

**Table 1**

| Protein Expression As Determined By Western Blot Analysis | | | |
|---|---|---|---|
| Treatment | Calcitriol at 10⁻⁷M | Calcitriol at 10⁻⁹M | Seocalcitol at 10⁻⁹M |
| Catalase | 170.21±56.01* | 131.34±26.60* | 162.68±39.88* |
| MnSOD | 103.58±14.29 | 99.44±20.95 | 113.48±20.56 |
| Cu/ZnSOD | 99.72±10.81 | 103.85±17.81 | 101.27±26.41 |
| GPX | 102.18±9.51 | 97.93±17.67 | 81.85±37.45 |

| | | | |
|---|---|---|---|
| Values represent the average percent intensity of the protein band relative to the control ± the standard deviation. Significant differences as compared to control: * = p<0.05. | | | |

### Example 3

The enzymatic activity of catalase was determined using assay kits from Cayman Chemical Co., Ann Arbor, MI, according to manufacturer's directions. The assay utilized the peroxidatic function of CAT for determination of enzymatic activity, based on the reaction of the enzyme with methanol in the presence of an optimal concentration of H₂O₂. The formaldehyde produced was measured spectrophotometrically with 4-amino-3-hydrazino-5mercapto-1,2,4-triazole (Purpald) as the chromagen. Purpald specifically forms a bicyclic heterocycle with aldehydes, which upon oxidation changes from colorless to a purple color. The absorbance was read at 540 nm using a spectrophotometric plate reader. Results were calculated from standard curve from known samples and by using the provided equations. From that, the enzymatic activity of CAT was derived as nmol/min/ml CAT. Samples were done in triplicate. A one-tail t-test assuming equal variance was performed to determine significance. Results are shown in Table 2.

**Table 2**

| Enzymatic Activity of Catalase | | | | | |
|---|---|---|---|---|---|
| Treatment | Control | Calcitriol at 10⁻⁷M | Calcitriol at 10⁻⁹M | Seocalcitol at 10⁻⁹M | Analog V at 10⁻⁹M |
| Catalase nmol/min/ml | 11.20±0.43 | 12.24±0.80 | 12.51±0.42* | 13.99±0.99** | 13.93±0.53** |

| | | | | | |
|---|---|---|---|---|---|
| Values represent the mean ± the standard deviation of nmol/min/ml CAT. Significant differences compared to control: * = p<0.05 and ** = p<0.01. | | | | | |

### Example 4

Total RNA was isolated using RNAqueous® kit from Ambion Inc. (Austin, TX, USA). Briefly, treated cells were trypsinized (at 37°C in the oven), and homogenized by a hand held electric homogenizer (at 4°C). Cell lysates were then subjected to total RNA isolation according to protocol provided by RNAqueous® kit and stored at -80°C. The RNA received was purified and concentrated to ∼1000 ng/ul using ethanol precipitation. The RNA was resuspended in DEPC water and DNAse I treated to remove residual DNA. The DNA-free™ DNAse Treatment kit from Ambion (cat #1906) was used for DNAse I treatment according to the manufacturer's directions. The Purified RNA was quantitated using the NanoDrop spectrophotometer and checked for integrity using the Agilent 2100 Bioanalyzer, following the RNA nano chip kit (cat#5067-1511) protocol.

Quantitative PCR. Quantitative PCR was performed to determine RNA expression changes for calcitriol at 10⁻⁷ M (n=5) and 10⁻⁹ M (n=5), and seocalcitol at 10⁻⁷ M (n=3) and 10⁻⁹ M (n=3) treated cells relative to control (n=5) for 24 hours. Reverse transcriptase reactions were performed using Super Script II® First-Strand Synthesis System for RT-PCR (Invitrogen # 11904-018) according to manufacturer's directions. For each sample reaction, 1µg of RNA was added to DEPC Treated & Nuclease free H2O (Fisher # BP561-1) to a final volume of 11.4uL. A Master Mix was made using 1.5µL10 mM dNTPs, 1.5µL random hexamers and 0.6µL Oligo dT primers per reaction (sample). 3.6uL of the Master Mix was added to each sample. Samples were incubated at 68°C for 10 minutes and then brought down to 4°C for at least 1 minute using a GeneAmp PCR System 9700 (Applied Biosystems). A portion (0.25X) of the above reaction was removed and used as a minus RT reaction (Negative Control containing No Super Script II® Reverse Transcriptase). Using the same Super Script II® Reverse Transcriptase kit, a master mix containing 3µL of 10X RT buffer, 6µL of 25mM MgCl2, 3µL 0.1M DTT and 1.5µL RNAse Inhibitor was made. A portion (0.25X) was removed and 0.375µL H2O per sample was added for the minus RT Master Mix. 3.75µL was added to each minus RT sample. To the remainder of the Master Mix, 1.125µL Super Script II® Reverse Transcriptase per sample was added for the Plus RT Master Mix. 11.25µL was added to each Plus RT sample. All reactions were then incubated at 42°C for 1hour, boiled at 95°C for 5 minutes, and brought down to 4°C using a GeneAmp PCR System 9700. The samples were then diluted 1 part RT reaction to 29 Parts TE buffer pH=8.0 (Ambion # 9858) to create a stock of cDNA for experimentation.

Primers and 5' nuclease assay probes were designed based on selected sequences using Primer ExpressTM v3.0 with standard factory defaults (Applied Biosystems Primer Express® Tutorial for Real Time Quantitative PCR Primer and Probe Design Tutorial). Minor groove Binding probes (ABI Custom Oligo Synthesis Factory) were ordered from ABI. All oligos were reconstituted with DEPC H2O to 100µM stock concentration, and then diluted with DEPC H2O to a 5µM working stock concentration. TaqMan® Fast Universal PCR Master Mix (2X) (Applied Biosystems # 4352042) was used for quantitative PCR reactions according to the manufacturer's directions. Final Primer concentration was 300µM each and final Probe concentration was 200µM (determined optimal from earlier experiments). 3.2µl of Plus RT and minus RT reactions were used for quantitative PCR reactions in a final volume of 20uL. All positive reactions were done in triplicate, and negative controls were performed singly. Standard qPCR conditions were used as described in the TaqMan® Fast Universal Master Mix (Applied Biosystems, 95.0°C for 20 seconds, and 40-50 cycles of 95°C for 03 seconds then 60°C for 30 seconds) at 20µL final volume. Samples were run on an ABI Prism 7500 Fast Real Time PCR System using ABI SDS Software v1.3.

All samples were run singularly against each primer/probe set to determine what sample should be used for the Standard Curve. Standard curves were generated using serial dilutions of RNA from experimental samples showing the lowest CT (cycle threshold) for the most targets. The low CT sample was re-reverse transcribed and a 1:10 serial dilution would be used as the standard curve for all primer/probe sets. Cycle threshold values were then used to calculate an input amount by using the standard curve and mean input amounts. Values were normalized to Cyclophilin A (Peptidylprolyl isomerase A) levels as determined by quantitative PCR. Inductions were calculated from each of the lowest sample's normalized value. The Dunnett's test was performed on the null hypothesis where the mean of each treatment group is equal to the mean of their common control group at the 99% confidence interval (alpha=0.01). Results are shown in Table 3.

**Table 3**

| Gene Expression Values As Determined By Quantitative PCR | | | | | | |
|---|---|---|---|---|---|---|
| Treatment | CAT | Cu/ZnSOD | MnSOD | VDR | PTHrP | TGFb |
| Control | 1.86±0.11 | 1.11±0.02 | 1.28±0.05 | 2.09±0.19 | 1.35±0.09 | 2.26±0.18 |
| Seocalcitol 10⁻⁷ M | 2.66±0.08** | 1.17±0.01 | 1.11±0.05 | 1.99±0.09 | 1.20±0.07 | 1.89±0.34 |
| Seocalcitol 10⁻⁹ M | 2.55±0.13** | 1.20±0.07 | 1.16±0.05 | 1.45±0.10 | 1.30±0.02 | 2.02±0.32 |
| Calcitriol 10⁻⁷ M | 2.60±0.11** | 1.18±0.03 | 1.05±0.03** | 1.66±0.09 | 1.16±0.05 | 1.95±0.11 |
| Calcitriol 10⁻⁹ M | 2.13±0.06 | 1.16±0.02 | 1.15±0.04 | 2.26±0.27 | 1.36±0.11 | 2.19±0.12 |

| | | | | | | |
|---|---|---|---|---|---|---|
| Values represent the means ± the standard errors of the means. ** represents a significant difference as compared to control at the 99% confidence interval. | | | | | | |

## Claims

1. A vitamin D for use in preventing or treating a disease or condition caused by catalase deficiency in an animal that can benefit from an increase in the production of catalase;
wherein the vitamin D is administered to the animal in a catalase production increasing amount of vitamin D;
wherein the animal is an aging companion animal;
wherein the vitamin D is one or more of calcitriol (1α,25-dihydroxyvitamin D₃), 25(OH) vitamin D₃, 20-Epi-1α,25(OH)₂D₃ and 1α,24(R)(OH)₂D₃; and
wherein the disease or condition is acatalasemia

2. A vitamin D as the sole active ingredient for use in preventing or treating a disease or condition caused by catalase deficiency in an animal that can benefit from an increase in the production of catalase;
wherein the vitamin D is administered to the animal in a catalase production increasing amount of vitamin D;
wherein the animal is an aging companion animal;
wherein the vitamin D is one or more of calcitriol (1α,25-dihydroxyvitamin D₃), 25(OH) vitamin D₃, 20-Epi-1α,25(OH)₂D₃ and 1α,24(R)(OH)₂D₃; and
wherein the disease or condition is hepatoma.

3. The vitamin D for use according to claim 1 or 2 wherein the vitamin D is administered in a composition formulated as a pet food composition or a dietary supplement.

4. The vitamin D for use according to claim 3 wherein the composition is a food composition comprising 15% to 50% protein, 5% to 40% fat, 5% to 10% ash content, and having a moisture content of 5% to 20%.

5. A composition for use in preventing or treating a disease or condition caused by catalase deficiency in an aging companion animal that can benefit from an increase in the production of catalase, the composition comprising a biologically acceptable medium in which is disposed one or more forms of vitamin D and at least one additional agent increasing catalase production or activity, or reducing or eliminating reactive oxygen species in the animal;
wherein the vitamin D is one or more of calcitriol (1α,25-dihydroxyvitam D₃), 25(OH) vitamin D₃, 20-Epi-1α,25(OH)₂D₃ and 1α,24(R)(OH)₂D₃; and
wherein the disease or condition is acatalasemia.

6. The composition for use of claim 5 comprising the vitamin D in amounts sufficient to provide from 1 ng/kg body weight to 10 mg/kg body weight.

7. The composition for use of claim 5 or 6 wherein the additional agent is one or more antioxidants.

8. The composition for use of any of claims 5 to 7 formulated as a pharmaceutical or nutraceutical composition comprising one or more one or more pharmaceutically or nutraceutically acceptable carriers, diluents, or excipients.

9. The composition for use of any of claims 5 to 8 formulated as a pet food composition or a dietary supplement.

10. The composition for use of claim 9 wherein the composition is a food composition further comprising 15% to 50% protein, 5% to 40% fat, 5% to 10% ash content, and having a moisture content of 5% to 20%.

11. The composition for use of claim 8 or 9 further comprising one or more probiotics, prebiotics, or a combination thereof.

## Patentansprüche

1. Vitamin D zur Verwendung beim Verhindern oder Behandeln einer Krankheit oder Störung, die durch Katalasemangel in einem Tier verursacht wird, das von einer gesteigerten Produktion von Katalase profitieren kann;
wobei das Vitamin D dem Tier in einer die Katalaseproduktion steigernden Menge an Vitamin D verabreicht wird;
wobei das Tier ein alterndes Haus- oder Nutztier ist;
wobei das Vitamin D eines oder mehrere von Calcitriol (1α,25-Dihydroxyvitamin D₃), 25(OH) Vitamin D₃, 20-Epi-1α,25(OH)₂D₃ und 1α,24(R)(OH)₂D₃ ist; und
wobei die Krankheit oder Störung Akatalasämie ist.

2. Vitamin D als einziger Wirkstoffbestandteil zur Verwendung beim Verhindern oder Behandeln einer Krankheit oder Störung, die durch Katalasemangel in einem Tier verursacht wird, das von einer gesteigerten Produktion von Katalase profitieren kann;
wobei das Vitamin D dem Tier in einer die Katalaseproduktion steigernden Menge an Vitamin D verabreicht wird;
wobei das Tier ein alterndes Haus- oder Nutztier ist;
wobei das Vitamin D eines oder mehrere von Calcitriol (1α,25-Dihydroxyvitamin D₃), 25(OH) Vitamin D₃, 20-Epi-1α,25(OH)₂D₃ und 1α,24(R)(OH)₂D₃ ist; und
wobei die Krankheit oder Störung ein Hepatom ist.

3. Vitamin D zur Verwendung nach Anspruch 1 oder 2, wobei das Vitamin D in einer Zusammensetzung verabreicht wird, die als eine Tierfutterzusammensetzung oder eine Nahrungsergänzung formuliert ist.

4. Vitamin D zur Verwendung nach Anspruch 3, wobei die Zusammensetzung eine Nahrungszusammensetzung ist, die von 15 % bis 50 % Protein, von 5 % bis 40 % Fett, von 5 % bis 10 % Aschegehalt umfasst, und einen Feuchtigkeitsgehalt von 5 % bis 20 % aufweist.

5. Zusammensetzung zur Verwendung beim Verhindern oder Behandeln einer Krankheit oder Störung, die durch Katalasemangel in einem alternden Haus- oder Nutztier verursacht wird, das von einer gesteigerten Produktion von Katalase profitieren kann, wobei die Zusammensetzung ein biologisch akzeptables Medium umfasst, in dem eine oder mehrere Formen von Vitamin D und wenigstens ein weiterer Wirkstoff angeordnet sind, der die Katalaseproduktion oder -aktivität steigert oder reaktive Sauerstoffspezies in dem Tier reduziert oder eliminiert;
wobei das Vitamin D eines oder mehrere von Calcitriol (1α,25-Dihydroxyvitamin D₃), 25(OH) Vitamin D₃, 20-Epi-1α,25(OH)₂D₃ und 1α,24(R)(OH)₂D₃ ist; und wobei die Krankheit oder Störung Akatalasämie ist.

6. Zusammensetzung zur Verwendung nach Anspruch 5, umfassend das Vitamin D in Mengen, die ausreichen, um 1 ng/kg Körpergewicht bis 10 mg/kg Körpergewicht bereitzustellen.

7. Zusammensetzung zur Verwendung nach Anspruch 5 oder 6, wobei der weitere Wirkstoff ein oder mehrere Antioxidationsmittel ist.

8. Zusammensetzung zur Verwendung nach einem der Ansprüche 5 bis 7, die als eine pharmazeutische oder nutrazeutische Zusammensetzung formuliert ist, die einen oder mehrere pharmazeutisch oder neutrazeutisch akzeptable Träger, Verdünnungsmittel oder Hilfsstoffe umfasst.

9. Zusammensetzung zur Verwendung nach einem der Ansprüche 5 bis 8, die als eine Tierfutterzusammensetzung oder eine Nahrungsergänzung formuliert ist.

10. Zusammensetzung zur Verwendung nach Anspruch 9, wobei die Zusammensetzung eine Nahrungszusammensetzung ist, die ferner von 15 % bis 50 % Protein, von 5 % bis 40 % Fett, von 5 % bis 10 % Aschegehalt umfasst, und einen Feuchtigkeitsgehalt von 5 % bis 20 % aufweist.

11. Zusammensetzung zur Verwendung nach Anspruch 8 oder 9, ferner umfassend ein oder mehrere Probiotika, Präbiotika oder eine Kombination davon.

## Revendications

1. Vitamine D destinée à être utilisée dans la prévention ou le traitement d'une maladie ou affection causée par une carence en catalase chez un animal qui peut profiter d'une augmentation de la production de catalase ;
où la vitamine D est administrée à l'animal en une quantité de vitamine D augmentant la production de catalase ;
dans laquelle l'animal est un animal de compagnie vieillissant ;
où la vitamine D est un ou plusieurs parmi la calcitriol (1α,25-dihydroxyvitamine D₃), la 25(OH) vitamine D₃, le 20-Epi-1α,25(OH)₂D₃ et le 1α,24(R)(OH)₂D₃ ; et
dans laquelle la maladie ou affection est l'acatalasémie.

2. Vitamine D en tant que principe actif unique, destinée à être utilisée dans la prévention ou le traitement d'une maladie ou affection causée par une carence en catalase chez un animal qui peut profiter d'une augmentation de la production de catalase ;
où la vitamine D est administrée à l'animal en une quantité de vitamine D augmentant la production de catalase ;
dans laquelle l'animal est un animal de compagnie vieillissant ;
où la vitamine D est un ou plusieurs parmi la calcitriol (1α,25-dihydroxyvitamine D₃), la 25(OH) vitamine D₃, le 20-Epi-1α,25(OH)₂D₃ et le 1α,24(R)(OH)₂D₃ ; et
dans laquelle la maladie ou affection est un hépatome.

3. Vitamine D utilisable selon la revendication 1 ou 2, où la vitamine D est administrée dans une composition formulée en tant composition alimentaire pour animaux de compagnie ou complément diététique.

4. Vitamine D utilisable selon la revendication 3, dans laquelle la composition est une composition alimentaire comprenant 15 % à 50 % de protéine, 5 % à 40 % de matière grasse, 5 % à 10 % de teneur en cendres, et possédant une teneur en humidité de 5 % à 20 %.

5. Composition utilisable dans la prévention ou le traitement d'une maladie ou affection causée par une carence en catalase chez un animal de compagnie vieillissant qui peut profiter d'une augmentation de la production de catalase, la composition comprenant un support biologiquement acceptable dans lequel sont disposés une ou plusieurs formes de vitamine D et au moins un agent supplémentaire augmentant la production ou activité de catalase, ou réduisant ou éliminant les espèces d'oxygène réactives chez l'animal ;
dans laquelle la vitamine D est un ou plusieurs parmi la calcitriol (1α,25-dihydroxyvitamine D₃), la 25(OH) vitamine D3, le 20-Epi-1α,25(OH)₂D₃ et le 1α,24(R)(OH)₂D₃ ; et dans laquelle la maladie ou affection est l'acatalasémie.

6. Composition utilisable selon la revendication 5, comprenant la vitamine D en des quantités suffisantes pour fournir de 1 ng/kg de poids corporel à 10 mg/kg de poids corporel.

7. Composition utilisable selon la revendication 5 ou 6, dans laquelle l'agent supplémentaire est un ou plusieurs antioxydants.

8. Composition utilisable selon l'une quelconque des revendications 5 à 7, formulée en tant composition pharmaceutique ou nutriceutique comprenant un ou plusieurs supports, diluants ou excipients acceptables sur le plan pharmaceutique ou nutriceutique.

9. Composition utilisable selon l'une quelconque des revendications 5 à 8, formulée en tant composition alimentaire pour animaux de compagnie ou complément diététique.

10. Composition utilisable selon la revendication 9, où la composition est une composition alimentaire comprenant en outre 15 % à 50 % de protéine, 5 % à 40 % de matière grasse, 5 % à 10 % de teneur en cendres, et possédant une teneur en humidité de 5 % à 20 %.

11. Composition utilisable selon la revendication 8 ou 9, comprenant en outre un ou plusieurs probiotiques, prébiotiques, ou une combinaison de ceux-ci.
